(19) Europäisches Patentamt
European Patent Office
Office européen des brevets

(11) **EP 4 317 206 A1**

(12) **EUROPEAN PATENT APPLICATION**
published in accordance with Art. 153(4) EPC

(43) Date of publication:
**07.02.2024 Bulletin 2024/06**

(21) Application number: **21932636.0**

(22) Date of filing: **01.11.2021**

(51) International Patent Classification (IPC):
**C08F 8/42** (2006.01)   **A61K 49/12** (2006.01)
**A61K 49/14** (2006.01)   **C08B 37/08** (2006.01)

(86) International application number:
**PCT/CN2021/127915**

(87) International publication number:
**WO 2022/199028 (29.09.2022 Gazette 2022/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.03.2021 CN 202110303742**

(71) Applicants:
• **SUZHOU ZHECI PHARMACEUTICAL
TECHNOLOGY CO., LTD.
Suzhou, Jiangsu 215011 (CN)**

• **Southern Medical University
Guangzhou, Guangdong 510515 (CN)**

(72) Inventors:
• **SHEN, Zheyu
Suzhou, Jiangsu 215011 (CN)**
• **LU, Yudie
Suzhou, Jiangsu 215011 (CN)**

(74) Representative: **Hafner & Kohl PartmbB
Schleiermacherstraße 25
90491 Nürnberg (DE)**

(54) **GADOLINIUM CHELATE, PREPARATION METHOD THEREFOR, AND APPLICATION THEREOF**

(57)   A gadolinium chelate, a preparation method therefor, and an application thereof. The gadolinium chelate is a complex formed by chelation of gadolinium ions and macromolecules. The macromolecules comprise any one of or a copolymer or mixture of two or more of carboxylic acid-containing high-molecular polymers, amino-containing high-molecular polymers, hydroxyl-containing high-molecular polymers, polyester high-molecular polymers, polyether high-molecular polymers, polyamide high-molecular polymers, proteins, polypeptides, and polysaccharides. The gadolinium ions and the macromolecules form the gadolinium chelate by means of chelation. The gadolinium chelate is a water-soluble macromolecular drug, can improve a longitudinal relaxation rate $r_1$ and reduce a ratio of $r_2/r_1$, has good water-solubility and high stability, and meanwhile, can exhibit a good imaging effect in a short period of time and shorten an MRI time.

EP 4 317 206 A1

**Description**

**TECHNICAL FIELD**

The present disclosure relates to the technical field of biomedical materials, and in particular to a gadolinium chelate, a preparation method and application thereof.

**BACKGROUND**

Magnetic resonance imaging (MRI) can be used for detecting small or specific lesions sensitively, thus reducing the rates of missed diagnosis or erroneous diagnosis. As a chemical agent capable of improving the quality of MRI images, an MRI contrast agent has become an important supplement to MRI examinations. $T_1$ (longitudinal relaxation time) and $T_2$ (transverse relaxation time) are important tissue feature parameters of MRI. When paramagnetic substances (such as gadolinium, iron and manganese) in a sample approach hydrogen atoms in resonance, the magnetic field where protons are located can be effectively changed, the tissue relaxation times $T_1$ and $T_2$ can be shortened, signals in an image enhancement region are thus enhanced, and the signal-to-noise ratio ($\Delta$SNR) of the tissue can be improved. Using contrast agents to improve the contrast ratio of MRI tissue exactly takes advantage of this function. The range of clinical application of MRI techniques is also greatly widened thanks to the addition of MRI contrast agents, and the MRI techniques have a very important application value in the field of clinical diagnosis.

Clinically, $T_1$-weighted imaging, $T_2$-weighted imaging, and $T_2$*-weighted imaging are the most commonly used imaging sequences to obtain tissue images. Correspondingly, MRI contrast agents are mainly classified into two types according to their enhancement characteristics: a positive contrast agent ($T_1$ contrast agent) and a negative contrast agent ($T_2$ contrast agent). Since the $T_2$ contrast agent has the features such as darker imaging and likely generated magnetic susceptibility artifacts, while the $T_1$ contrast agent has a more prominent effect on brightening lesion areas in images, the $T_1$ contrast agent exhibits a very obvious advantage in soft tissue detection. A relaxation rate including longitudinal relaxation rate $r_1$, and transverse relaxation rate $r_2$, is one of the key evaluation indexes to measure the performance of the MRI contrast agents. Generally, if an $r_1$ value is larger and an $r_2/r_1$ value is smaller, it is more conducive to $T_1$-weighted imaging, and the signal-to-noise ratio of images is higher under the condition of the same dose; conversely, if the $r_2$ value is larger and the $r_2/r_1$ value is larger, it is more conducive to $T_2$-weighted imaging. At present, commercially available $T_1$ contrast agents are mainly gadolinium-based contrast agents, including cyclic chelates and linear chelates. The cyclic chelates include, for example, Gadavist® (Gd-BT-DO3A), Dotarem® (Gd-DOTA) and ProHance® (Gd-HP-DO3A), etc., and the linear chelates include, for example, Magnevist® (Gd-DTPA), Omniscan® (Gd-DTPA-BMA), Primovist® (Gd-EOB-DTPA) and Multihance® (Gd-BOTPA), etc. These commercially available gadolinium-based contrast agents are micromolecular, which exhibit very limited relaxation properties, and provide $r_1$ values in the range of 4-7 $mM^{-1}\ s^{-1}$.

Since both linear and macrocyclic gadolinium-based contrast agents cause trace gadolinium deposition in the brain and other tissues, if high doses of gadolinium-based contrast agents are used in clinical scanning to enhance the contrast between normal tissues and diseased tissues, it may result in potential risks such as nephrotoxicity in some patient populations. Currently, in order to take into account higher-sensitivity lesion detection and dose limitation, the contrast agents for clinical diagnosis are usually dosed at a gram level. Decreasing the dose of a contrast agent used is a very desirable way to improve the signal-to-noise ratio of an image, because a compound having a high relaxation can be detected at a low dose, or present a greater contrast to a compound having a low relaxation at an equivalent dose, making a contrast effect better.

**SUMMARY**

The present disclosure aims to solve at least one of the technical problems existing in the prior art. In view of this, the present disclosure provides a gadolinium chelate, which has an extremely high longitudinal relaxation rate $r_1$ and an extremely low $r_2/r_1$ ratio, good water solubility and high stability, and can exhibit a good imaging effect in a short time and shorten an MRI time.

The present disclosure further provides a preparation method and an application of the gadolinium chelate.

Specifically, the technical solution used in the present disclosure is as follows.

In a first aspect, the present disclosure provides a gadolinium chelate. The gadolinium chelate is a complex formed by chelation of gadolinium ions and a macromolecule, wherein the macromolecule includes any one of or a copolymer or mixture of two or more of a carboxylic acid -containing high-molecular polymer, an amino-containing high-molecular polymer, a hydroxyl-containing high-molecular polymer, a polyester high-molecular polymer, a polyether high-molecular polymer, a polyamide high-molecular polymer, a protein, a polypeptide, or a polysaccharide.

According to the first aspect, the gadolinium chelate of the present disclosure has at least the following beneficial effects.

In the present disclosure, the gadolinium ions and the macromolecule chelate to form the gadolinium chelate that is a water-soluble macromolecular drug. Compared with the network crosslinked structures or core-shell structures formed by wrapping gadolinium ions or gadolinium oxides with macromolecules in the related art, the longitudinal relaxation rate $r_1$ of the gadolinium chelate can be increased, the $r_2/r_1$ ratio thereof can be decreased, and the gadolinium chelate have a good water solubility and high stability, and exhibit a good imaging effect in a short time and shorten an MRI time.

In some embodiments of the present disclosure, a molecular weight of the macromolecule is 1,000-1,000,000, and suitable macromolecules may be selected according to a specific circumstance.

In some embodiments of the present disclosure, the carboxylic acid-containing high-molecular polymer includes any one or more selected from the group consisting of polyacrylic acid, polymaleic acid, polymethacrylic acid, poly(2-ethylacrylic acid), polyglutamic acid, and polyaspartic acid.

In some embodiments of the present disclosure, the amino-containing high-molecular polymer includes any one or more selected from the group consisting of polylysine, polyarginine, polyhistidine, and polyethyleneimine.

In some embodiments of the present disclosure, the hydroxyl-containing high-molecular polymer includes any one or more selected from the group consisting of polyserine, polythreonine, polytyrosine, and polyvinyl alcohol.

In some embodiments of the present disclosure, the polyester high-molecular polymer includes any one or more selected from the group consisting of polylactic acid, polyglycolic acid, polycaprolactone, and poly(2-hydroxyethyl methacrylate).

In some embodiments of the present disclosure, the polyether high-molecular polymer includes any one or more selected from the group consisting of polyethylene glycol, polypropylene glycol, and polytetramethylene glycol.

In some embodiments of the present disclosure, the polyamide high-molecular polymer includes any one or more selected from the group consisting of poly glutamine, polyasparagine, polyacrylamide, and polymethacrylamide.

In some embodiments of the present disclosure, the protein includes any one or more selected from the group consisting of a human protein, an animal protein, a plant protein, and a recombinant protein.

In some embodiments of the present disclosure, the polypeptide includes any one or more selected from the group consisting of an RGD peptide and a β-amyloid polypeptide.

In some embodiments of the present disclosure, the polysaccharide includes any one or more selected from the group consisting of chitosan and sodium alginate.

In some embodiments of the present disclosure, the gadolinium ions are trivalent gadolinium ions.

In a second aspect, the present disclosure provides a preparation method of the above-mentioned gadolinium chelate, comprising: subjecting gadolinium ions and a macromolecule to a chelation reaction to obtain the gadolinium chelate.

More specifically, the preparation method of the above-mentioned gadolinium chelate, comprising: mixing a gadolinium ion solution with a macromolecular solution to perform a chelation reaction, so as to obtain a gadolinium chelate.

In some embodiments of the present disclosure, a concentration of the gadolinium ion solution is 10-1000 mM, preferably 50-250 mM.

In some embodiments of the present disclosure, a concentration of the macromolecular solution is 0.1-50 mg/mL, preferably 2.0-10 mg/mL.

In some embodiments of the present disclosure, a volume ratio of the macromolecular solution to the gadolinium ion solution is (1-1000): 1, preferably (25-50):1.

In some embodiments of the present disclosure, both the gadolinium ion solution and the macromolecular solution are aqueous solutions.

In some embodiments of the present disclosure, the gadolinium ion solution includes any one or more selected from the group consisting of a gadolinium nitrate solution, a gadolinium fluoride solution, a gadolinium chloride solution, a gadolinium bromide solution and a gadolinium iodide solution, preferably the gadolinium nitrate or gadolinium chloride solution.

In some embodiments of the present disclosure, a pH of a reaction solution obtained by mixing the gadolinium ion solution and the macromolecular solution is 2.0-12.0, preferably about 10.

In some embodiments of the present disclosure, the reaction is conducted at a temperature of 25°C-100°C, preferably 100°C.

In some embodiments of the present disclosure, the reaction is lasts for more than 10 min, preferably 30-120 min.

In a third aspect, the present disclosure provides an application of the above-mentioned gadolinium chelate in the preparation of a magnetic resonance imaging contrast agent (MRI contrast agent).

Compared with the prior art, the present disclosure has the following beneficial effects.

The gadolinium chelate of the present disclosure has an extremely high $r_1$ value (>80 mM$^{-1}$ s$^{-1}$, 1.5 T; ≥29 mM$^{-1}$ s$^{-1}$, 3.0 T), which is much higher than that ($r_1$ = 4-7 mM$^{-1}$ s$^{-1}$, 1.5 T) of the MRI contrast agent commonly used in clinical practice, and is also higher than that (>50 mM$^{-1}$ s$^{-1}$, 1.5 T) of the MRI contrast agent obtained by coating a gadolinium oxide with a high molecular polymer, and that (14 mM$^{-1}$ s$^{-1}$, 3.0 T) of a polymer network microsphere contrast agent prepared from gadolinium ions and a polymer used in the related art, and the gadolinium chelate has an extremely low $r_2/r_1$ ratio ($r_2/r_1$<2.0, 1.5 T). As a novel MRI contrast agent, the gadolinium chelate of the present disclosure can not only significantly improve the signal-to-noise ratio of MRI images, but also has the advantages of excellent water solubility, ultra-low release rate of gadolinium (III) ions within 7 days, being stable and effective, etc. Moreover, the gadolinium chelate of the present disclosure has a rapid imaging effect at a low dose, a shortened imaging time, and strong practicability.

## BRIEF DESCRIPTION OF DRAWINGS

FIG. 1 is a chart showing an MRI relaxation rate of Gd-PAA of Example 1;

FIG. 2 shows a gadolinium ion release rate of Gd-PAA of Example 1 under the condition of pH = 7.4 for 1-7 days;

FIG. 3 shows T$_1$-weighted MRI images of Gd-PAA of Example 1, a commercially available contrast agent Gadavist and pure water and corresponding MRI signal intensities thereof;

FIG. 4 is an infrared absorption spectrogram of Gd-PAA prepared in Example 1;

FIG. 5 is a MRI image showing a tumor-bearing mice after injecting with Gd-PAA prepared in Example 1;

FIG. 6 is a chart showing a relaxation rate of Gd-PASP of Example 2;

FIG. 7 shows a gadolinium ion release rate of Gd-PASP of Example 2 under the condition of pH = 7.4 for 1-7 days;

FIG. 8 shows T$_1$-weighted MRI images of Gd-PASP of Example 2, a commercially available contrast agent Gadavist and pure water and corresponding MRI signal intensities thereof;

FIG. 9 is a chart showing a relaxation rate of Gd-HPMA of Example 3;

FIG. 10 is a chart showing a relaxation rate of Gd-PMAA of Example 4;

FIG. 11 is a chart showing a relaxation rate of Gd-PEAA of Example 5;

FIG. 12 is a chart showing a relaxation rate of Gd-$\gamma$-PGA of Example 6;

FIG. 13 is a chart showing a relaxation rate of Gd-$\epsilon$-PL of Example 7;

FIG. 14 is a chart showing a relaxation rate of Gd-PLR of Example 8;

FIG. 15 is a chart showing a relaxation rate of Gd-PLH of Example 9;

FIG. 16 is a chart showing a relaxation rate of Gd-PEI of Example 10;

FIG. 17 is a chart showing a relaxation rate of Gd-PSer of Example 11;

FIG. 18 is a chart showing a relaxation rate of Gd-PThr of Example 12;

FIG. 19 is a chart showing a relaxation rate of Gd-PTyr of Example 13;

FIG. 20 is a chart showing a relaxation rate of Gd-PVA of Example 14;

FIG. 21 is a chart showing a relaxation rate of Gd-PLA of Example 15;

FIG. 22 is a chart showing a relaxation rate of Gd-PGA of Example 16;

FIG. 23 is a chart showing a relaxation rate of Gd-PCL of Example 17;

FIG. 24 is a chart showing a relaxation rate of Gd-PHEMA of Example 18;

FIG. 25 is a chart showing a relaxation rate of Gd-PEG of Example 19;

FIG. 26 is a chart showing a relaxation rate of Gd-PPG of Example 20;

FIG. 27 is a chart showing a relaxation rate of Gd-PTMG of Example 21;

FIG. 28 is a chart showing a relaxation rate of Gd-PolyQ of Example 22;

FIG. 29 is a chart showing a relaxation rate of Gd-PHEA of Example 23;

FIG. 30 is a chart showing a relaxation rate of Gd-PAM of Example 24;

FIG. 31 is a chart showing a relaxation rate of Gd-PMAM of Example 25;

FIG. 32 is a chart showing a relaxation rate of Gd-HSA of Example 26;

FIG. 33 is a chart showing a relaxation rate of Gd-BSA of Example 27;

FIG. 34 is a chart showing a relaxation rate of Gd-RGD of Example 28;

FIG. 35 is a chart showing a relaxation rate of Gd-A$\beta$ of Example 29;

FIG. 36 is a chart showing a relaxation rate of Gd-CS of Example 30;

FIG. 37 is a chart showing a relaxation rate of Gd-SA of Example 31;

FIG. 38 is a chart showing a relaxation rate of Gd-PAA-PLA of Example 32;

FIG. 39 is a chart showing a relaxation rate of Gd-PGA-PEG of Example 33;

FIG. 40 is a chart showing a relaxation rate of Gd-PAA/PASP of Example 34; and

FIG. 41 is a chart showing a relaxation rate of Gd-$\gamma$-PGA/PASP of Example 35.

## DETAILED DESCRIPTION

The technical solutions of the present disclosure will be further described in conjunction with the following specific examples.

### Example 1

Preparation of gadolinium chelate (Gd-PAA)

40 mL of polyacrylic acid (molecular weight ($M_W$) = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PAA finally.

Characterization and performance tests were performed on a sample provided in Example 1. The gadolinium recovery rate of the sample prepared in Example 1 was calculated, and it was higher than 90%, indicating that a chelating effect was good and the utilization rate of raw materials was high. The sample prepared in Example 1, and the commercially available contrast agents (Magnevist, and Gadavist), and a gadolinium nitrate solution were each prepared into at least 5 aqueous solutions of different concentrations, in vitro imaging was performed by means of 1.5 T and 3.0 T clinical MRI systems and a 7.0 T small animal MRI system, and longitudinal relaxation times and transverse relaxation times ($T_1$,

$T_2$) were determined. A longitudinal relaxation rate and a transverse relaxation rate ($r_1$, $r_2$) were calculated by the following formula (c represents the concentration of a magnetic substance in the contrast agent, T represents the relaxation time, where i = 1 or 2). The results are shown in Table 1.

$$r_i = \frac{\Delta(1/T_i)}{\Delta c}$$

Table 1 Synthesis of Gd-PAA and MRI characterization results

| Sample | Gadolinium recovery rate (%) | $H_0$(T) | $r_1$ (mM$^{-1}$ s$^{-1}$) | $r_2$ (mM$^{-1}$ s$^{-1}$) | $r_2/r_1$ |
|---|---|---|---|---|---|
| Gd-PAA | 95.5 | 1.5 | 82.76 ± 0.81 | 92.46 ± 0.58 | 1.12 ± 0.00 |
| | | 3.0 | 56.23 ± 1.69 | 86.58 ± 1.18 | 1.54 ± 0.03 |
| | | 7.0 | 17.18 | 92.55 | 5.39 |
| Gadavist | - | 3.0 | 4.60 ± 0.09 | 4.90 ± 0.04 | 1.07 ± 0.03 |
| | | 7.0 | 3.77 | 5.56 | 1.47 |
| Magnevist | - | 3.0 | 4.88 ± 0.12 | 5.00 ± 0.10 | 1.03 ± 0.03 |
| | | 7.0 | 3.68 | 5.38 | 1.46 |
| Gd(NO$_3$)$_3$ | - | 3.0 | 11.39 ± 0.25 | 12.04 ± 0.31 | 1.06 ± 0.006 |
| | | 7.0 | 8.92 | 12.90 | 1.45 |

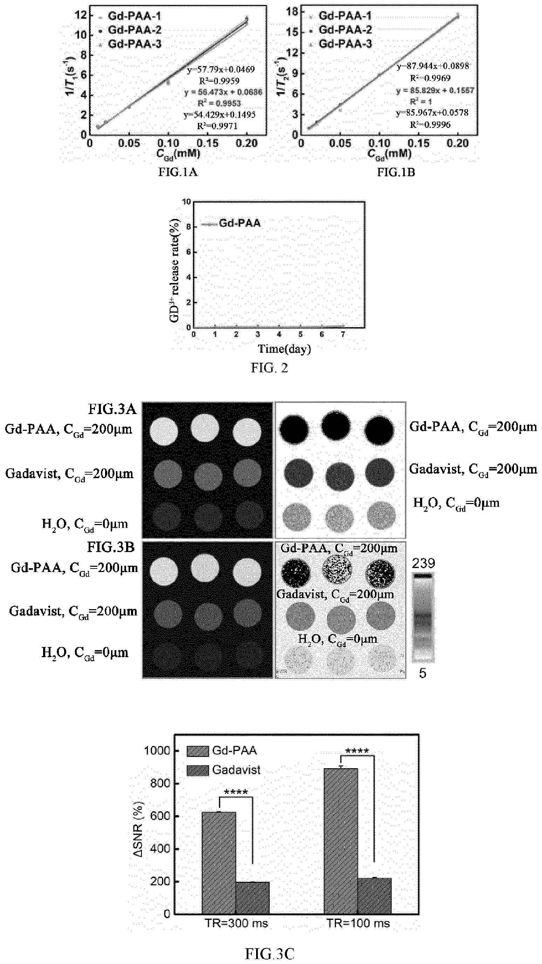

Specifically, FIG. 1 is a chart showing a relaxation rate of the samples Gd-PAA of Example 1 when used as an MRI contract agent (three samples Gd-PAA-1, Gd-PAA-2 and Gd-PAA-3 were subjected to a parallel test for three times), and in the relationship that $1/T_i$ changes with the gadolinium concentration, a slope of the fitted straight line is the $T_i$ relaxation rate (i = 1 or 2). It could be seen that in the 3.0 T clinical MRI scanning system (Philips, Ingenia), the longitudinal relaxation rate of the gadolinium chelate Gd-PAA is 56.23 ± 1.69 mM$^{-1}$ s$^{-1}$, which is more than ten times the $r_1$ value ($r_1$ = 4-7 mM$^{-1}$ s$^{-1}$) of the commercially available gadolinium-based contrast agent, and significantly higher than that of a highly toxic free gadolinium ion solution. In addition, the Gd-PAA has $r_1$ value tested by the 1.5 T MRI scanning system of 82.76 ± 0.81 mM$^{-1}$ s$^{-1}$, and also has an extremely low $r_2/r_1$ ratio (1.12 ± 0.00), indicating that the MRI contrast effect is particularly excellent.

FIG. 2 shows a gadolinium (III) ion release rate of the sample Gd-PAA under the condition of pH = 7.4 for 1-7 days. The sample Gd-PAA hardly releases ions (<0.2%), indicating that the sample provided in Example 1 has high stability when used as the MRI contrast agent.

On the basis of the $r_1$ value, the $r_2/r_1$ ratio in Table 1 and the gadolinium ion release rate, taking the sample Gd-PAA as a typical representative, T1-weighted MRI images of Gd-PAA, the commercially available contrast agent Gadavist and the pure water under a 7.0 T magnetic field are shown in FIG. 3, wherein TR = 300 ms and TE = 6.5 ms in (a), TR = 100 ms and TE = 6.5 ms in (b), and (c) shows the relative MRI signal intensities of Gd-PAA, Gadavist and the pure water, ****p<0.0001. The results show that the MRI signal intensity of Gd-PAA is much higher than that of the commercial contrast agent Gadavist.

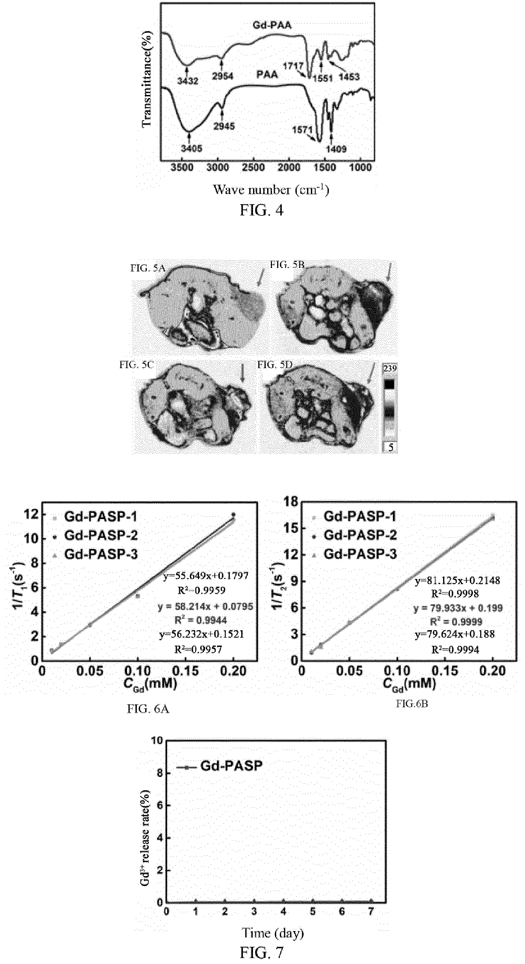

Referring to FIG. 4, it is an infrared absorption spectrogram of the typical sample Gd-PAA. A strong and broad absorption peak of polyacrylic acid at 3405 cm$^{-1}$ is an O-H stretching vibration peak, while an O-H stretching vibration peak of the sample Gd-PAA moves to 3432 cm$^{-1}$, the absorption peak is narrowed, and its intensity is weakened, indicating that an O-H and a hydrogen bond at this position breaks and reacts with Gd$^{3+}$. The polyacrylic acid has antisymmetric and symmetric stretching vibration absorption peaks of carboxyl groups at 1571 cm$^{-1}$ and 1409 cm$^{-1}$. The sample Gd-PAA has a new C=O stretching vibration absorption peak at 1717 cm$^{-1}$, indicating that oxygen and gadolinium ions are coordinate in the carboxyl group of the polyacrylic acid, thus proving the formation of the gadolinium chelate Gd-PAA.

FIG. 5 show MRI images of a tumor-bearing mice suffered from tail vein injection with the Gd-PAA chelate in different time periods (with an injection dose of 5 mg/kg). Wherein (a) is an MRI image obtained before tail vein injection, (b) is an MRI image obtained at 15 minutes after tail vein injection, (c) is an MRI image obtained at 30 minutes after tail vein injection, and (d) is an MRI image obtained at 45 minutes after injection of the Gd-PAA chelate. It can be seen that an MRI signal of the tumor site is the strongest at 30 minutes after intravenous injection, indicating that this macromolecular chelate can not only remarkably improve the imaging contrast ratio of tumor tissue, but also has a shorter imaging time, and is conducive to clinical promotion.

**Example 2**

Preparation of gadolinium chelate (Gd-PASP)

40 mL of polyaspartic acid ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PASP finally.

Characterization and performance tests were performed on a sample provided in Example 2. The gadolinium recovery rate of the sample prepared in Example 2 was higher than 90%, indicating that the utilization rate of raw materials was high. The sample prepared in Example 2 was prepared into at least 5 aqueous solutions of different concentrations, in vitro imaging was performed by means of the 3.0 T clinical MRI system and the 7.0 T MRI system, and a longitudinal relaxation time and a transverse relaxation time ($T_1$, $T_2$) were determined. The calculated results of the longitudinal relaxation rate and transverse relaxation rate ($r_1$, $r_2$) are shown in Table 2.

Table 2 Synthesis of Gd-PASP and MRI characterization results

| Sample | Gadolinium recovery rate (%) | $H_0$ (T) | $r_1$ (mM$^{-1}$ s$^{-1}$) | $r_2$ (mM$^{-1}$ s$^{-1}$) | $r_2/r_1$ |
|---|---|---|---|---|---|
| Gd-PASP | 91.3% | 3.0 | $56.70 \pm 1.34$ | $80.23 \pm 0.80$ | $1.42 \pm 0.05$ |
| | | 7.0 | 19.60 | 76.84 | 3.92 |

Specifically, FIG. 6 is a chart showing a relaxation rate of the samples Gd-PASP of Example 2 when used as an MRI contract agent (three samples Gd-PASP-1, Gd-PASP-2 and Gd- PASP-3 were subjected to a parallel test for three times), and in the relationship that $1/T_i$ changes with the gadolinium concentration, a slope of the fitted straight line is the $T_i$ relaxation rate (i = 1 or 2). It can be seen that in the 3.0 T clinical MRI scanning system (Philips, Ingenia), the $r_1$ value of the gadolinium chelate Gd-PASP is $56.70 \pm 1.34$ mM$^{-1}$ s$^{-1}$, which is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PASP also has a lower $r_2/r_1$ ratio, showing a better MRI contrast effect.

FIG. 7 shows a gadolinium ion release rate of the sample Gd-PASP under the condition of pH = 7.4 for 1-7 days. The sample Gd-PASP hardly releases ions (<0.1%), indicating that Gd-PASP has good stability when used as the MRI contrast agent.

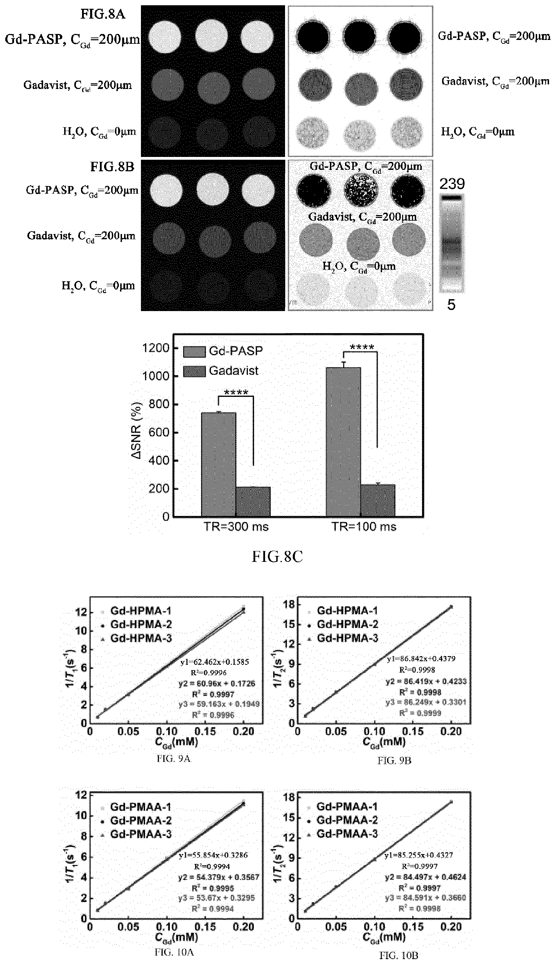

FIG. 8 shows $T_1$-weighted MRI images of Gd-PASP, the commercially available contrast agent Gadavist and pure water and corresponding relative MRI signal intensities thereof. The magnetic field was 7.0 T (Bruker, PharmaScan 70/16 US). Wherein TR = 300 ms and TE = 6.5 ms in (a), TR = 100 ms and TE = 6.5 ms in (b), and (c) shows the relative MRI signal intensities of Gd-PASP, Gadavist and the pure water, ****p<0.0001. The results show that the MRI signal intensity of Gd-PASP is much higher than that of Gadavist.

**Example 3**

Preparation of gadolinium chelate (Gd-HPMA)

40 mL of polymaleic acid ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysisto obtain a product labeled as Gd-HPMA finally.

Characterization and performance tests were performed on a sample provided in Example 3. The gadolinium recovery rate of the sample prepared in Example 3 was higher, indicating that the utilization rate of raw materials was high. The sample prepared in Example 3 was prepared into at least 5 aqueous solutions with different concentrations, in vitro imaging was performed by means of the 3.0 T clinical MRI system, and a longitudinal relaxation time and a transverse relaxation time ($T_1$, $T_2$) thereof were measured. The calculated results of the longitudinal relaxation rate and transverse relaxation rate ($r_1$, $r_2$) are shown in Table 3.

Table 3 Synthesis of Gd-HPMA and MRI characterization results

| Sample | Gadolinium recovery rate (%) | $H_0(T)$ | $r_1$ (mM$^{-1}$ s$^{-1}$) | $r_2$ (mM$^{-1}$ s$^{-1}$) | $r_2/r_1$ |
|---|---|---|---|---|---|
| Gd-HPMA | 94.4% | 3.0 | 60.86 ± 1.65 | 86.50 ± 0.31 | 1.42 ± 0.03 |

Specifically, FIG. 9 is a chart showing a relaxation rate of the samples Gd-HPMA of Example 3 when used as an MRI contract agent (three samples Gd-HPMA-1, Gd-HPMA-2 and Gd-HPMA-3 were subjected to a parallel test for three times), and in the relationship that $1/T_i$ changes with the gadolinium concentration, a slope of the fitted straight line is the $T_i$ relaxation rate (i = 1 or 2). It can be seen that in the 3.0 T clinical MRI scanning system (Philips, Ingenia), the $r_1$ value of the gadolinium chelate Gd-HPMA is 60.86 ± 1.65 mM$^{-1}$ s$^{-1}$, which is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-HPMA also has a lower $r_2/r_1$ ratio, showing a better MRI contrast effect.

**Example 4**

Preparation of gadolinium chelate (Gd-PMAA)

40 mL of polymethacrylic acid ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under magnetic stirring. After being cooled to the room temperature, the solution was purified by means of membrane dialysis to obtain a product labeled as Gd-PMAA finally.

Characterization and performance tests were performed on a sample provided in Example 4. The gadolinium recovery rate of the sample prepared in Example 4 was higher, indicating that the utilization rate of raw materials was high. The sample prepared in Example 4 was prepared into at least 5 aqueous solutions with different concentrations, in vitro imaging was performed by means of the 3.0 T clinical MRI system, and a longitudinal relaxation time and a transverse relaxation time ($T_1$, $T_2$) thereof were measured. The calculated results of the longitudinal relaxation rate and transverse relaxation rate ($r_1$, $r_2$) are shown in Table 4.

Table 4 Synthesis of Gd-PMAA and MRI characterization results

| Sample | Gadolinium recovery rate (%) | $H_0$ (T) | $r_1$ (mM$^{-1}$ s$^{-1}$) | $r_2$ (mM$^{-1}$ s$^{-1}$) | $r_2/r_1$ |
|---|---|---|---|---|---|
| Gd-PMAA | 93.6% | 3.0 | 54.63 ± 1.11 | 84.78 ± 0.41 | 1.55 ± 0.02 |

Specifically, FIG. 10 is a chart showing a relaxation rate of the samples Gd-PMAA of Example 4 when used as an MRI contract agent (three samples Gd-PMAA-1, Gd-PMAA-2 and Gd-PMAA-3 were subjected to a parallel test for three times), and in the relationship that $1/T_i$ changes with the gadolinium concentration, a slope of the fitted straight line is the $T_i$ relaxation rate (i = 1 or 2). It can be seen that in the 3.0 T clinical MRI scanning system (Philips, Ingenia), the $r_1$ value of the gadolinium chelate Gd-PMAA is 54.63 ± 1.11 mM$^{-1}$ s$^{-1}$, which is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PMAA also has a lower $r_2/r_1$ ratio, showing a better MRI contrast effect.

**Example 5**

Preparation of gadolinium chelate (Gd-PEAA)

40 mL of poly(2-ethylacrylic acid) ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysisto obtain a product labeled as Gd-PEAA finally.

Characterization and performance tests were performed on a sample provided in Example 5. The gadolinium recovery rate of the sample prepared in Example 5 was higher, indicating that the utilization rate of raw materials was high. The sample prepared in Example 5 was prepared into at least 5 aqueous solutions with different concentrations, in vitro imaging was performed by means of the 3.0 T clinical MRI system, and a longitudinal relaxation time and a transverse relaxation time ($T_1$, $T_2$) thereof were measured. The calculated results of the longitudinal relaxation rate and transverse relaxation rate ($r_1$, $r_2$) are shown in Table 5.

Table 5 Synthesis of Gd-PEAA and MRI characterization results

| Sample | Gadolinium recovery rate (%) | $H_0$ (T) | $r_1$ (mM$^{-1}$ s$^{-1}$) | $r_2$ (mM$^{-1}$ s$^{-1}$) | $r_2/r_1$ |
|---|---|---|---|---|---|
| Gd-PEAA | 93.3% | 3.0 | 53.13 $\pm$ 0.64 | 84.00 $\pm$ 1.39 | 1.58 $\pm$ 0.01 |

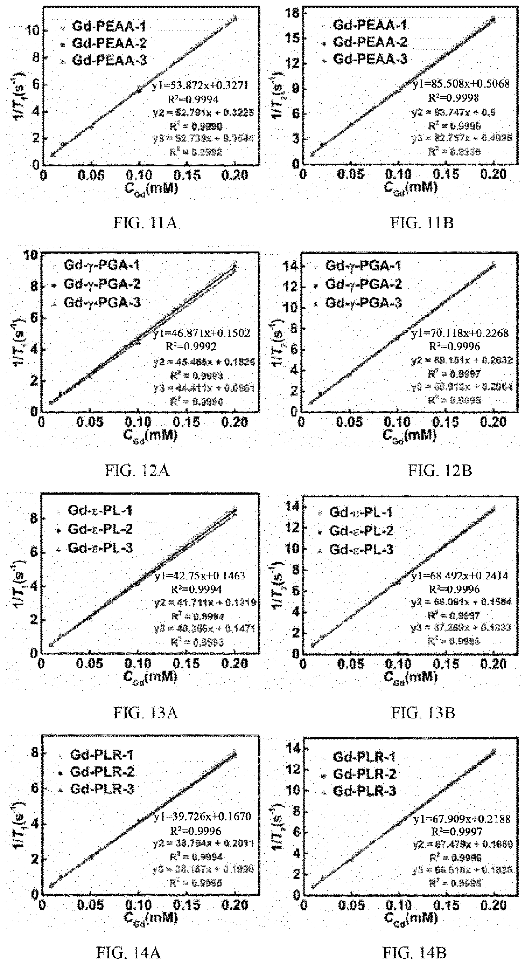

Specifically, FIG. 11 is a chart showing a relaxation rate of the samples Gd-PEAA of Example 5 when used as an MRI contract agent (three samples Gd-PEAA-1, Gd-PEAA-2 and Gd-PEAA-3 were subjected to a parallel test for three times), and in the relationship that $1/T_i$ changes with the gadolinium concentration, a slope of the fitted straight line is the $T_i$ relaxation rate (i = 1 or 2). It can be seen that in the 3.0 T clinical MRI scanning system (Philips, Ingenia), the $r_1$ value of the gadolinium chelate Gd-PEAA is 53.13 $\pm$ 0.64 mM$^{-1}$ s$^{-1}$, which is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PEAA also has a lower $r_2/r_1$ ratio, showing a better MRI contrast effect.

**Example 6**

Preparation of gadolinium chelate (Gd-γ-PGA)

40 mL of polyglutamic acid ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysisto obtain a product labeled as Gd-γ-PGA finally.

Characterization and performance tests were performed on a sample provided in Example 6. The gadolinium recovery rate of the sample prepared in Example 6 was higher, indicating that the utilization rate of raw materials was high. The sample prepared in Example 6 was prepared into at least 5 aqueous solutions with different concentrations, in vitro imaging was performed by means of the 3.0 T clinical MRI system, and a longitudinal relaxation time and a transverse relaxation time ($T_1$, $T_2$) thereof were measured. The calculated results of the longitudinal relaxation rate and transverse relaxation rate ($r_1$, $r_2$) are shown in Table 6.

Table 6 Synthesis of Gd-γ-PGA and MRI characterization results

| Sample | Gadolinium recovery rate (%) | $H_0$(T) | $r_1$ (mM$^{-1}$ s$^{-1}$) | $r_2$ (mM$^{-1}$ s$^{-1}$) | $r_2/r_1$ |
|---|---|---|---|---|---|
| Gd-γ-PGA | 82.4% | 3.0 | 45.59 $\pm$ 1.23 | 69.39 $\pm$ 0.64 | 1.52 $\pm$ 0.03 |

Specifically, FIG. 12 is a chart showing a relaxation rate of the samples Gd-γ-PGA of Example 6 when used as an MRI contract agent (three samples Gd-γ-PGA-1, Gd-γ-PGA-2 and Gd-γ-PGA-3 were subjected to a parallel test for three times), and in the relationship that $1/T_i$ changes with the gadolinium concentration, a slope of the fitted straight line is the $T_i$ relaxation rate (i = 1 or 2). It can be seen that in the 3.0 T clinical MRI scanning system (Philips, Ingenia), the $r_1$ value of the gadolinium chelate Gd-γ-PGA is 45.59 $\pm$ 1.23 mM$^{-1}$ s$^{-1}$, which is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-γ-PGA also has a lower $r_2/r_1$ ratio, showing a better MRI contrast effect.

**Example 7**

Preparation of gadolinium chelate (Gd-ε-PL)

40 mL of polylysine ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-$\varepsilon$-PL finally.

Characterization and performance tests were performed on a sample provided in Example 7. The gadolinium recovery rate of the sample prepared in Example 7 was higher, indicating that the utilization rate of raw materials was high. The sample prepared in Example 7 was prepared into at least 5 aqueous solutions with different concentrations, in vitro imaging was performed by means of the 3.0 T clinical MRI system, and a longitudinal relaxation time and a transverse relaxation time ($T_1$, $T_2$) thereof were measured. The finally calculated results of the longitudinal relaxation rate and transverse relaxation rate ($r_1$, $r_2$) are shown in Table 7.

Table 7 Synthesis of Gd-$\varepsilon$-PL and MRI characterization results

| Sample | Gadolinium recovery rate (%) | $H_0$ (T) | $r_1$ (mM$^{-1}$ s$^{-1}$) | $r_2$ (mM$^{-1}$ s$^{-1}$) | $r_2/r_1$ |
|---|---|---|---|---|---|
| Gd-$\varepsilon$-PL | 88.6% | 3.0 | 41.61 $\pm$ 1.20 | 67.95 $\pm$ 0.62 | 1.63 $\pm$ 0.03 |

Specifically, FIG. 13 is a chart showing a relaxation rate of the samples Gd-$\varepsilon$-PL of Example 7 when used as an MRI contract agent (three samples Gd-$\varepsilon$-PL-1, Gd-$\varepsilon$-PL-2 and Gd-$\varepsilon$-PL-3 were subjected to a parallel test for three times), and in the relationship that $1/T_i$ changes with the gadolinium concentration, a slope of the fitted straight line is the $T_i$ relaxation rate (i = 1 or 2). It can be seen that in the 3.0 T clinical MRI scanning system (Philips, Ingenia), the $r_1$ value of the gadolinium chelate Gd-$\varepsilon$-PL is 41.61 $\pm$ 1.20 mM$^{-1}$ s$^{-1}$, which is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-$\varepsilon$-PL also has a lower $r_2/r_1$ ratio, showing a better MRI contrast effect.

**Example 8**

Preparation of gadolinium chelate (Gd-PLR)

40 mL of polyarginine ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PLR finally.

Characterization and performance tests were performed on a sample provided in Example 8. The gadolinium recovery rate of the sample prepared in Example 8 was higher, indicating that the utilization rate of raw materials was high. The sample prepared in Example 8 was prepared into at least 5 aqueous solutions with different concentrations, in vitro imaging was performed by means of the 3.0 T clinical MRI system, and a longitudinal relaxation time and a transverse relaxation time ($T_1$, $T_2$) thereof were measured. The finally calculated results of the longitudinal relaxation rate and transverse relaxation rate ($r_1$, $r_2$) are shown in Table 8.

Table 8 Synthesis of Gd-PLR and MRI characterization results

| Sample | Gadolinium recovery rate (%) | $H_0$ (T) | $r_1$ (mM$^{-1}$ s$^{-1}$) | $r_2$ (mM$^{-1}$ s$^{-1}$) | $r_2/r_1$ |
|---|---|---|---|---|---|
| Gd-PLR | 87.8% | 3.0 | 38.90 $\pm$ 0.78 | 67.54 $\pm$ 0.80 | 1.74 $\pm$ 0.02 |

Specifically, FIG. 14 is a chart showing a relaxation rate of the samples Gd-PLR of Example 8 when used as an MRI contract agent (three samples Gd-PLR-1, Gd-PLR-2 and Gd-PLR-3 were subjected to a parallel test for three times), and in the relationship that $1/T_i$ changes with the gadolinium concentration, a slope of the fitted straight line is the $T_i$ relaxation rate (i = 1 or 2). It can be seen that in the 3.0 T clinical MRI scanning system (Philips, Ingenia), the $r_1$ value of the gadolinium chelate Gd-PLR is 38.90 $\pm$ 0.78 mM$^{-1}$ s$^{-1}$, which is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PLR also has a lower $r_2/r_1$ ratio, showing a better MRI contrast effect.

**Example 9**

Preparation of gadolinium chelate (Gd-PLH)

40 mL of polyhistidine ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under magnetic stirring. After being cooled to the room temperature, the solution was purified by means of membrane dialysis to obtain a product labeled as Gd-PLH finally.

Characterization and performance tests were performed on a sample provided in Example 9. The gadolinium recovery rate of the sample prepared in Example 9 was higher, indicating that the utilization rate of raw materials was high. The sample prepared in Example 9 was prepared into at least 5 aqueous solutions with different concentrations, in vitro imaging was performed by means of the 3.0 T clinical MRI system, and a longitudinal relaxation time and a transverse relaxation time ($T_1$, $T_2$) thereof were measured. The finally calculated results of the longitudinal relaxation rate and transverse relaxation rate ($r_1$, $r_2$) are shown in Table 9.

Table 9 Synthesis of Gd-PLH and MRI characterization results

| Sample | Gadolinium recovery rate (%) | $H_0$ (T) | $r_1$ (mM$^{-1}$ s$^{-1}$) | $r_2$ (mM$^{-1}$ s$^{-1}$) | $r_2/r_1$ |
|---|---|---|---|---|---|
| Gd-PLH | 90.2% | 3.0 | 45.46 $\pm$ 0.95 | 68.65 $\pm$ 0.39 | 1.51 $\pm$ 0.02 |

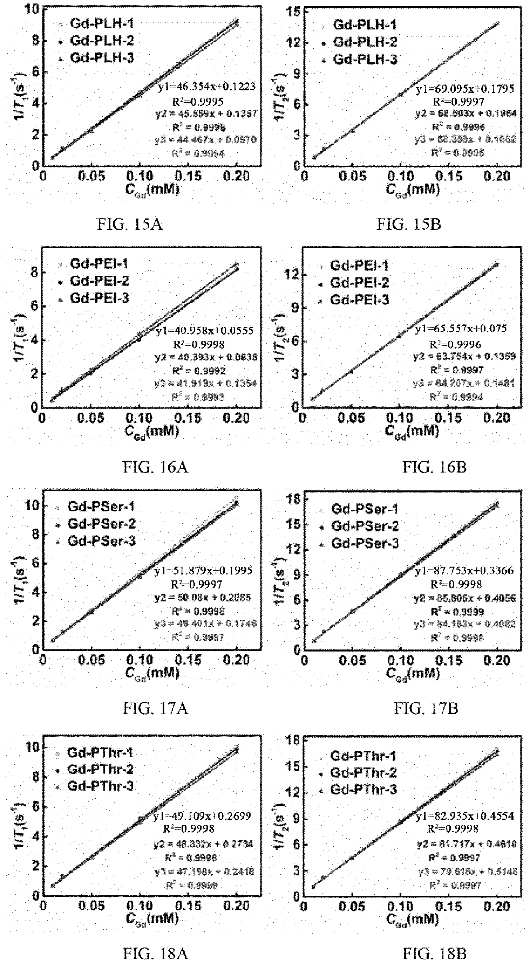

Specifically, FIG. 15 is a chart showing a relaxation rate of the samples Gd-PLH of Example 9 when used as an MRI contract agent (three samples Gd-PLH-1, Gd-PLH-2 and Gd-PLH-3 were subjected to a parallel test for three times), and in the relationship that $1/T_i$ changes with the gadolinium concentration, a slope of the fitted straight line is the $T_i$ relaxation rate (i = 1 or 2). It can be seen that in the 3.0 T clinical MRI scanning system (Philips, Ingenia), the $r_1$ value of the gadolinium chelate Gd-PLH is 45.46 $\pm$ 0.95 mM$^{-1}$ s$^{-1}$, which is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PLH also has a lower $r_2/r_1$ ratio, showing a better MRI contrast effect.

**Example 10**

Preparation of gadolinium chelate (Gd-PEI)

40 mL of polyethyleneimine ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PEI finally.

Characterization and performance tests were performed on a sample provided in Example 10. The gadolinium recovery rate of the sample prepared in Example 10 was higher than 90%, indicating that the utilization rate of raw materials was high. The sample prepared in Example 10 was prepared into at least 5 aqueous solutions with different concentrations, in vitro imaging was performed by means of the 3.0 T clinical MRI system, and a longitudinal relaxation time and a transverse relaxation time ($T_1$, $T_2$) thereof were measured. The calculated results of the longitudinal relaxation rate and transverse relaxation rate ($r_1$, $r_2$) are shown in Table 10.

Table 10 Synthesis of Gd-PEI and MRI characterization results

| Sample | Gadolinium recovery rate (%) | $H_0$ (T) | $r_1$ (mM$^{-1}$ s$^{-1}$) | $r_2$ (mM$^{-1}$ s$^{-1}$) | $r_2/r_1$ |
|---|---|---|---|---|---|
| Gd-PEI | 90.5% | 3.0 | 41.09$\pm$0.77 | 64.51 $\pm$ 0.94 | 1.57 $\pm$ 0.04 |

Specifically, FIG. 16 is a chart showing a relaxation rate of the samples Gd-PEI of Example 10 when used as an MRI contract agent (three samples Gd-PEI-1, Gd-PEI-2 and Gd-PEI-3 were subjected to a parallel test for three times), and in the relationship that 1/Ti changes with the gadolinium concentration, a slope of the fitted straight line is the $T_i$ relaxation rate (i = 1 or 2). It can be seen that in the 3.0 T clinical MRI scanning system (Philips, Ingenia), the $r_1$ value of the gadolinium chelate Gd-PEI is 41.09 $\pm$ 0.77 mM$^{-1}$ s$^{-1}$, which is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PEI also has a lower $r_2/r_1$ ratio, showing a better MRI contrast effect.

**Example 11**

Preparation of gadolinium chelate (Gd-PSer)

40 mL of poly serine ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PSer finally.

Characterization and performance tests were performed on a sample provided in Example 11. The gadolinium recovery rate of the sample prepared in Example 11 was 88.3%, indicating that the utilization rate of raw materials was higher. The sample prepared in Example 11 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PSer-1, Gd-PSer-2 and Gd-PSer-3 were subjected to a parallel test for three times). As shown in FIG. 17, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PSer is 50.45 $\pm$ 1.28 mM$^{-1}$ s$^{-1}$, the $r_2$ value is 85.90 $\pm$ 1.80 mM$^{-1}$ s$^{-1}$, and $r_2/r_1$ is 1.70 $\pm$ 0.01. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PSer also has a lower $r_2/r_1$ ratio, showing a better MRI contrast effect.

**Example 12**

Preparation of gadolinium chelate (Gd-PThr)

40 mL of polythreonine ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PThr finally.

Characterization and performance tests were performed on a sample provided in Example 12. The gadolinium recovery rate of the sample prepared in Example 12 was 88.3%, indicating that the utilization rate of raw materials was higher. The sample prepared in Example 12 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PThr-1, Gd-PThr-2 and Gd-PThr-3 were subjected to a parallel test for three times). As shown in FIG. 18, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PThr is 48.21 $\pm$ 0.96 mM$^{-1}$ s$^{-1}$, the $r_2$ value is 81.42 $\pm$ 1.68 mM$^{-1}$ s$^{-1}$, and $r_2/r_1$ is 1.69 $\pm$ 0.00. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PThr also has a lower $r_2/r_1$ ratio, showing a better MRI contrast effect.

**Example 13**

Preparation of gadolinium chelate (Gd-PTyr)

40 mL of polytyrosine ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PTyr finally.

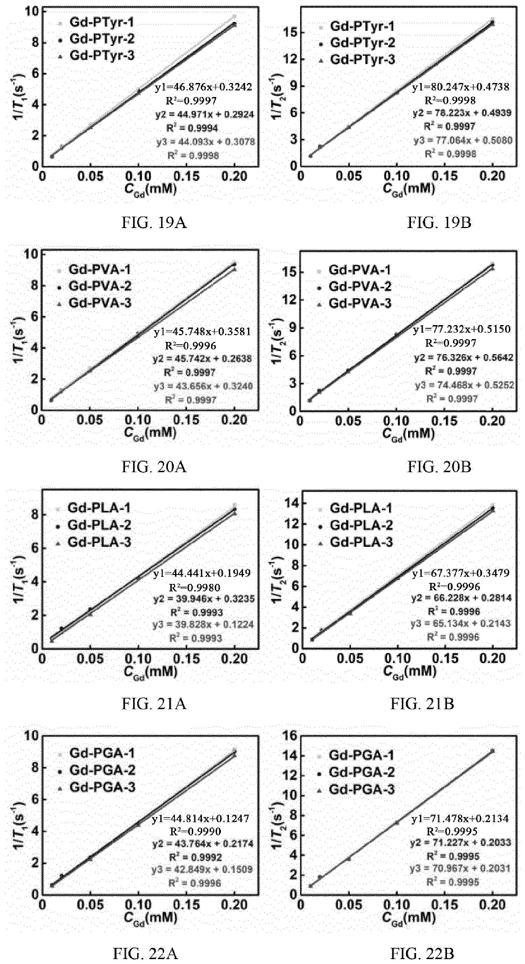

Characterization and performance tests were performed on a sample provided in Example 13. The gadolinium recovery rate of the sample prepared in Example 13 was 88.3%, indicating that the utilization rate of raw materials was higher. The sample prepared in Example 13 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PTyr-1, Gd-PTyr-2 and Gd-PTyr-3 were subjected to a parallel test for three times). As shown in FIG. 19, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PTyr is 45.31 $\pm$ 1.42 mM$^{-1}$ s$^{-1}$, the $r_2$ value is 78.51 $\pm$ 1.61 mM$^{-1}$ s$^{-1}$, and $r_2/r_1$ is 1.73 $\pm$ 0.02. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PTyr also has a lower $r_2/r_1$ ratio, showing a better MRI contrast effect.

## Example 14

Preparation of gadolinium chelate (Gd-PVA)

40 mL of polyvinyl alcohol ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under magnetic stirring. After being cooled to the room temperature, the solution was purified by means of membrane dialysis to obtain a product labeled as Gd-PVA finally.

Characterization and performance tests were performed on a sample provided in Example 14. The gadolinium recovery rate of the sample prepared in Example 14 was 88.3%, indicating that the utilization rate of raw materials was higher. The sample prepared in Example 14 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PVA-1, Gd-PVA-2 and Gd-PVA-3 were subjected to a parallel test for three times). As shown in FIG. 20, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PVA is 45.05 $\pm$ 1.21 mM$^{-1}$ s$^{-1}$, the $r_2$ value is 76.01 $\pm$ 1.41 mM$^{-1}$ s$^{-1}$, and $r_2/r_1$ is 1.69 $\pm$ 0.02. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PVA also has a lower $r_2/r_1$ ratio, showing a better MRI contrast effect.

## Example 15

Preparation of gadolinium chelate (Gd-PLA)

40 mL of polylactic acid ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PLA finally.

Characterization and performance tests were performed on a sample provided in Example 15. The gadolinium recovery rate of the sample prepared in Example 15 was 89.3%, indicating that the utilization rate of raw materials was higher. The sample prepared in Example 15 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PLA-1, Gd-PLA-2 and Gd-PLA-3 were subjected to a parallel test for three times). As shown in FIG. 21, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PLA is 40.41 $\pm$ 0.90 mM$^{-1}$ s$^{-1}$, the $r_2$ value is 66.25 $\pm$ 1.12 mM$^{-1}$ s$^{-1}$, and $r_2/r_1$ is 1.64 $\pm$ 0.02. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PLA also has a lower $r_2/r_1$ ratio, showing a better MRI contrast effect.

## Example 16

Preparation of gadolinium chelate (Gd-PGA)

40 mL of polyglycolic acid ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PGA finally.

Characterization and performance tests were performed on a sample provided in Example 16. The gadolinium recovery rate of the sample prepared in Example 16 was 90.9%, indicating that the utilization rate of raw materials was higher. The sample prepared in Example 16 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PGA-1, Gd-PGA-2 and Gd-PGA-3were subjected to a parallel test for three times). As shown in FIG. 22, the relationship of 1/Ti changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PGA is 43.81 $\pm$ 0.98 mM$^{-1}$ s$^{-1}$, the $r_2$ value is 71.22 $\pm$ 0.26 mM$^{-1}$ s$^{-1}$, and $r_2/r_1$ is 1.63 $\pm$ 0.03. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PGA also has a lower $r_2/r_1$ ratio, showing a better MRI contrast effect.

**Example 17**

Preparation of gadolinium chelate (Gd-PCL)

40 mL of polycaprolactone ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PCL finally.

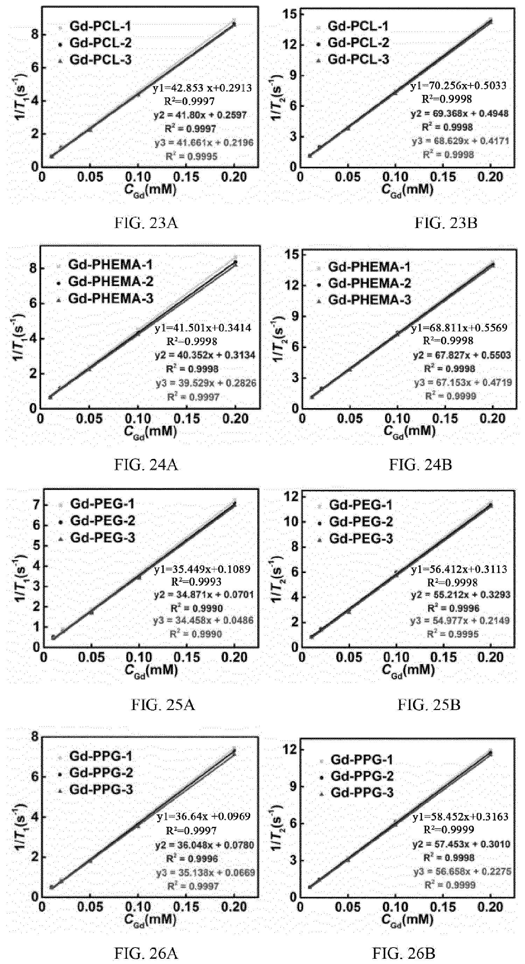

Characterization and performance tests were performed on a sample provided in Example 17. The gadolinium recovery rate of the sample prepared in Example 17 was 86.6%, indicating that the utilization rate of raw materials was higher. The sample prepared in Example 17 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PCL-1, Gd-PCL-2 and Gd-PCL-3 were subjected to a parallel test for three times). As shown in FIG. 23, the relationship of 1/T; changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PCL is 42.10 $\pm$ 0.65 mM$^{-1}$ s$^{-1}$, the $r_2$ value is 69.42 $\pm$ 0.81 mM$^{-1}$ s$^{-1}$, and $r_2/r_1$ is 1.65 $\pm$ 0.01. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PCL also has a lower $r_2/r_1$ ratio, showing a better MRI contrast effect.

**Example 18**

Preparation of gadolinium chelate (Gd-PHEMA)

40 mL of poly(2-hydroxyethyl methacrylate) ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PHEMA finally.

Characterization and performance tests were performed on a sample provided in Example 18. The gadolinium recovery rate of the sample prepared in Example 18 was 87.2%, indicating that the utilization rate of raw materials was higher. The sample prepared in Example 18 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PHEMA-1, Gd-PHEMA-2 and Gd-PHEMA-3 were subjected to a parallel test for three times). As shown in FIG. 24, the relationship of 1/$T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PHEMA is 40.46 $\pm$ 0.99 mM$^{-1}$ s$^{-1}$, the $r_2$ value is 67.93 $\pm$ 0.83 mM$^{-1}$ s$^{-1}$, and $r_2/r_1$ is 1.68 $\pm$ 0.02. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PHEMA also has a lower $r_2/r_1$ ratio,

showing a better MRI contrast effect.

**Example 19**

Preparation of gadolinium chelate (Gd-PEG)

40 mL of polyethylene glycol ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PEG finally.

Characterization and performance tests were performed on a sample provided in Example 19. The gadolinium recovery rate of the sample prepared in Example 19 was 94.5%, indicating that the utilization rate of raw materials was high. The sample prepared in Example 19 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PEG-1, Gd-PEG-2 and Gd-PEG-3 were subjected to a parallel test for three times). As shown in FIG. 25, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PEG is 34.93 $\pm$ 0.50 mM$^{-1}$ s$^{-1}$, the $r_2$ value is 55.53 $\pm$ 0.77 mM$^{-1}$ s$^{-1}$, and $r_2/r_1$ is 1.59 $\pm$ 0.01. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PEG also has a lower $r_2/r_1$ ratio, showing a better MRI contrast effect.

**Example 20**

Preparation of gadolinium chelate (Gd-PPG)

40 mL of polypropylene glycol ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PPG finally.

Characterization and performance tests were performed on a sample provided in Example 20. The gadolinium recovery rate of the sample prepared in Example 20 was 89.2%, indicating that the utilization rate of raw materials was higher. The sample prepared in Example 20 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PPG-1, Gd-PPG-2 and Gd-PPG-3 were subjected to a parallel test for three times). As shown in FIG. 26, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PPG is 35.94 $\pm$ 0.76 mM$^{-1}$ s$^{-1}$, the $r_2$ value is 57.52 $\pm$ 0.90 mM$^{-1}$ s$^{-1}$, and $r_2/r_1$ is 1.60 $\pm$ 0.01. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PPG also has a lower $r_2/r_1$ ratio, showing a better MRI contrast effect.

**Example 21**

Preparation of gadolinium chelate (Gd-PTMG)

40 mL of polytetramethylene glycol ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under magnetic stirring. After being cooled to the room temperature, the solution was purified by means of membrane dialysis to obtain a product labeled as Gd-PTMG finally.

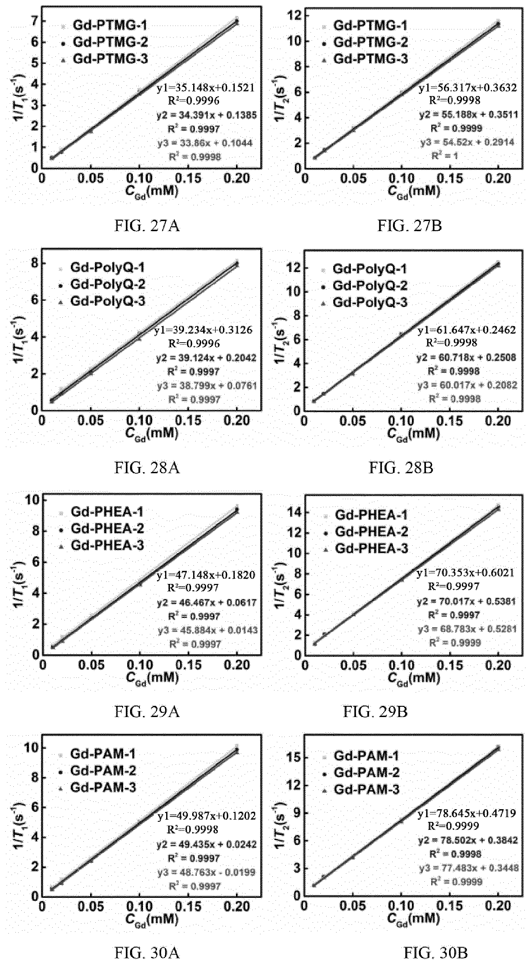

Characterization and performance tests were performed on a sample provided in Example 21. The gadolinium recovery rate of the sample prepared in Example 21 was 86.4%, indicating that the utilization rate of raw materials was higher. The sample prepared in Example 21 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PTMG-1, Gd-PTMG-2 and Gd-PTMG-3 were subjected to a parallel test for three times). As shown in FIG. 27, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PTMG is 34.47 $\pm$ 0.65 mM$^{-1}$ s$^{-1}$, the $r_2$ value is 55.34 $\pm$ 0.92 mM$^{-1}$ s$^{-1}$, and $r_2/r_1$ is 1.61 $\pm$ 0.00. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PTMG also has a lower $r_2/r_1$ ratio, showing a better MRI contrast effect.

## Example 22

Preparation of gadolinium chelate (Gd-PolyQ)

40 mL of polyglutamine ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PolyQ finally.

Characterization and performance tests were performed on a sample provided in Example 22. The gadolinium recovery rate of the sample prepared in Example 22 was 88.7%, indicating that the utilization rate of raw materials was higher. The sample prepared in Example 22 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PolyQ-1, Gd-PolyQ-2 and Gd-PolyQ-3 were subjected to a parallel test for three times). As shown in FIG. 28, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PolyQ is 39.05 $\pm$ 0.23 mM$^{-1}$ s$^{-1}$, the $r_2$ value is 60.79 $\pm$ 0.82 mM$^{-1}$ s$^{-1}$, and $r_2/r_1$ is 1.56 $\pm$ 0.01. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PolyQ also has a lower $r_2/r_1$ ratio, showing a better MRI contrast effect.

## Example 23

Preparation of gadolinium chelate (Gd-PHEA)

40 mL of polyasparagine ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PHEA finally.

Characterization and performance tests were performed on a sample provided in Example 23. The gadolinium recovery rate of the sample prepared in Example 23 was 91.2%, indicating that the utilization rate of raw materials was higher. The sample prepared in Example 23 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PHEA-1, Gd-PHEA-2 and Gd-PHEA-3 were subjected to a parallel test for three times). As shown in FIG. 29, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PHEA is 46.50 $\pm$ 0.63 mM$^{-1}$ s$^{-1}$, the $r_2$ value is 69.72 $\pm$ 0.83 mM$^{-1}$ s$^{-1}$, and $r_2/r_1$ is 1.50 $\pm$ 0.01. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PHEA also has a lower $r_2/r_1$ ratio, showing a better MRI contrast effect.

**Example 24**

Preparation of gadolinium chelate (Gd-PAM)

40 mL of polyacrylamide ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PAM finally.

Characterization and performance tests were performed on a sample provided in Example 24. The gadolinium recovery rate of the sample prepared in Example 24 was 90.6%, indicating that the utilization rate of raw materials was higher. The sample prepared in Example 24 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PAM-1, Gd-PAM-2 and Gd-PAM-3 were subjected to a parallel test for three times). As shown in FIG. 30, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PAM is 49.40 $\pm$ 0.61 mM$^{-1}$ s$^{-1}$, the $r_2$ value is 78.21 $\pm$ 0.63 mM$^{-1}$ s$^{-1}$, and $r_2/r_1$ is 1.58 $\pm$ 0.01. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PAM also has a lower $r_2/r_1$ ratio, showing a better MRI contrast effect.

**Example 25**

Preparation of gadolinium chelate (Gd-PMAM)

40 mL of polymethacrylamide ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PMAM finally.

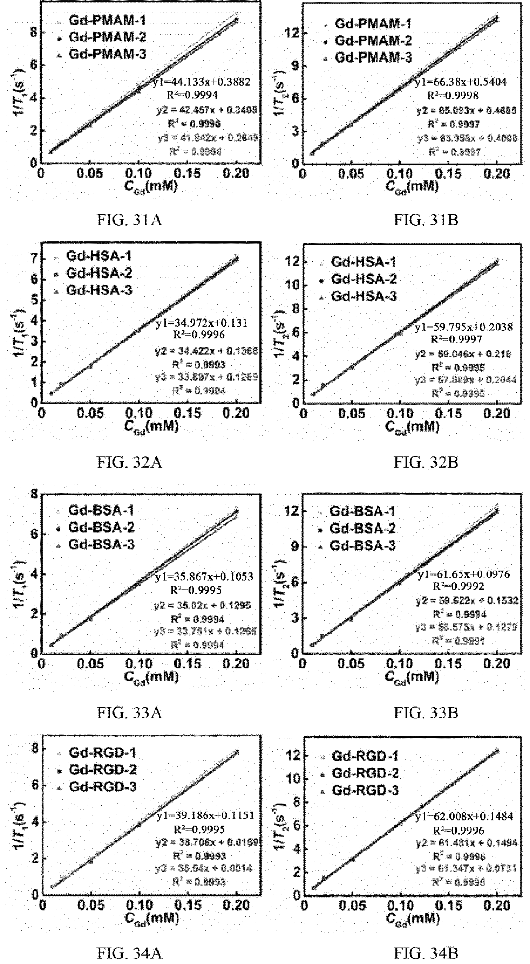

Characterization and performance tests were performed on a sample provided in Example 25. The gadolinium recovery rate of the sample prepared in Example 25 was 87.7%, indicating that the utilization rate of raw materials was higher. The sample prepared in Example 25 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PMAM-1, Gd-PMAM-2 and Gd-PMAM-3 were subjected to a parallel test for three times). As shown in FIG. 31, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PMAM is 42.81 $\pm$ 1.19 mM$^{-1}$ s$^{-1}$, the $r_2$ value is 65.14 $\pm$ 1.21 mM$^{-1}$ s$^{-1}$, and $r_2/r_1$ is 1.52 $\pm$ 0.02. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PMAM also has a lower $r_2/r_1$ ratio, showing a better MRI contrast effect.

**Example 26**

Preparation of gadolinium chelate (Gd-HSA)

40 mL of human serum albumin ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-HSA finally.

Characterization and performance tests were performed on a sample provided in Example 26. The gadolinium recovery rate of the sample prepared in Example 26 was 90.2%, indicating that the utilization rate of raw materials was higher. The sample prepared in Example 26 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-HSA-1, Gd-HSA-2 and Gd-HSA-3 were subjected to a parallel test for three times). As shown in FIG. 32, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-HSA is $34.43 \pm 0.54$ mM$^{-1}$ s$^{-1}$, the $r_2$ value is $59.92 \pm 1.57$ mM$^{-1}$ s$^{-1}$, and $r_2/r_1$ is $1.74 \pm 0.02$. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-HSA also has a lower $r_2/r_1$ ratio, showing a better MRI contrast effect.

**Example 27**

Preparation of gadolinium chelate (Gd-BSA)

40 mL of bovine serum albumin ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-BSA finally.

Characterization and performance tests were performed on a sample provided in Example 27. The gadolinium recovery rate of the sample prepared in Example 27 was 85.6%, indicating that the utilization rate of raw materials was higher. The sample prepared in Example 27 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-BSA-1, Gd-BSA-2 and Gd-BSA-3 were subjected to a parallel test for three times). As shown in FIG. 33, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-BSA is $34.88 \pm 1.06$ mM$^{-1}$ s$^{-1}$, the $r_2$ value is $59.92 \pm 1.57$ mM$^{-1}$ s$^{-1}$, and $r_2/r_1$ is $1.72 \pm 0.02$. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-BSA also has a lower $r_2/r_1$ ratio, showing a better MRI contrast effect.

**Example 28**

Preparation of gadolinium chelate (Gd-RGD)

40 mL of a RGD peptide ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-RGD finally.

Characterization and performance tests were performed on a sample provided in Example 28. The gadolinium recovery rate of the sample prepared in Example 28 was 82.0%, indicating that the utilization rate of raw materials was higher. The sample prepared in Example 28 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-RGD-1, Gd-RGD-2 and Gd-RGD-3 were subjected to a parallel test for three times). As shown in FIG. 34, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-RGD is $38.81 \pm 0.34$ mM$^{-1}$ s$^{-1}$, the $r_2$ value is $61.61 \pm 0.35$ mM$^{-1}$ s$^{-1}$, and $r_2/r_1$ is $1.59 \pm 0.01$. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-RGD also has a lower $r_2/r_1$ ratio, showing a better MRI contrast effect.

**Example 29**

Preparation of gadolinium chelate (Gd-A$\beta$)

40 mL of a $\beta$-amyloid polypeptide ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-Aβ finally.

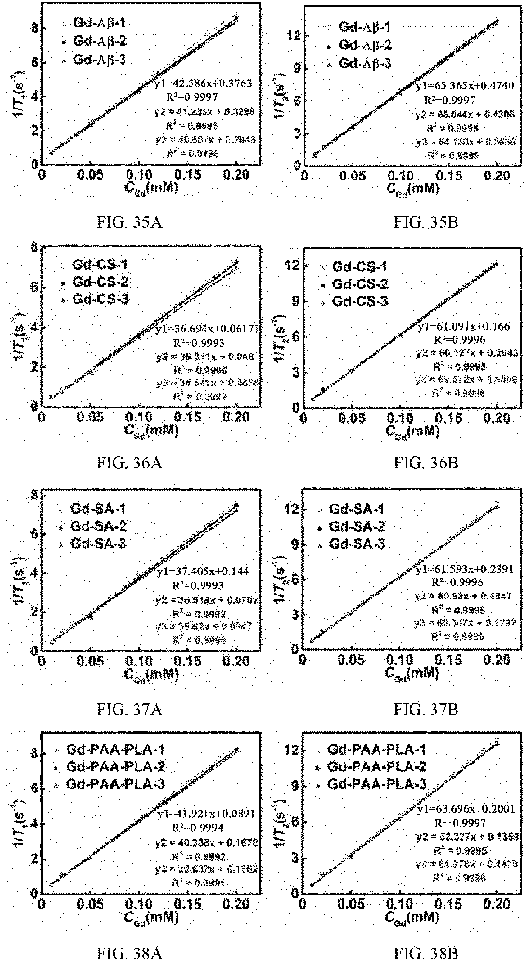

Characterization and performance tests were performed on a sample provided in Example 29. The gadolinium recovery rate of the sample prepared in Example 29 was 84.4%, indicating that the utilization rate of raw materials was higher. The sample prepared in Example 29 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-Aβ-1, Gd-Aβ-2 and Gd-Aβ-3 were subjected to a parallel test for three times). As shown in FIG. 35, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-Aβ is $41.47 \pm 1.01$ mM$^{-1}$ s$^{-1}$, the $r_2$ value is $64.85 \pm 0.64$ mM$^{-1}$ s$^{-1}$, and $r_2/r_1$ is $1.56 \pm 0.03$. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-Aβ also has a lower $r_2/r_1$ ratio, showing a better MRI contrast effect.

**Example 30**

Preparation of gadolinium chelate (Gd-CS)

40 mL of chitosan ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-CS finally.

Characterization and performance tests were performed on a sample provided in Example 30. The gadolinium recovery rate of the sample prepared in Example 30 was 83.6%, indicating that the utilization rate of raw materials was higher. The sample prepared in Example 30 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-CS-1, Gd-CS-2 and Gd-CS-3 were subjected to a parallel test for three times). As shown in FIG. 36, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-CS is $35.75 \pm 1.10$ mM$^{-1}$ s$^{-1}$, the $r_2$ value is $60.30 \pm 0.72$ mM$^{-1}$ s$^{-1}$, and $r_2/r_1$ is $1.69 \pm 0.03$. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-CS also has a lower $r_2/r_1$ ratio, showing a better MRI contrast effect.

**Example 31**

Preparation of gadolinium chelate (Gd-SA)

40 mL of sodium alginate ($M_w$ = 2000) solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-SA finally.

Characterization and performance tests were performed on a sample provided in Example 31. The gadolinium recovery rate of the sample prepared in Example 31 was 82.5%, indicating that the utilization rate of raw materials was higher. The sample prepared in Example 31 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, T2) thereof were measured (three samples Gd-SA-1, Gd-SA-2 and Gd-SA-3 were subjected to a parallel test for three times). As shown in FIG. 37, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-SA is $36.65 \pm 0.92$ mM$^{-1}$ s$^{-1}$, the $r_2$ value is $60.84 \pm 0.66$ mM$^{-1}$ s$^{-1}$, and $r_2/r_1$ is $1.67 \pm 0.03$. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-SA also has a lower $r_2/r_1$ ratio, showing a better MRI contrast effect.

## Example 32

Preparation of gadolinium chelate (Gd-PAA-PLA)

40 mL of polyacrylic acid-polylactic acid ($M_w$ = 2000) copolymer solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PAA-PLA finally.

Characterization and performance tests were performed on a sample provided in Example 32. The gadolinium recovery rate of the sample prepared in Example 32 was 88.6%, indicating that the utilization rate of raw materials was higher. The sample prepared in Example 32 was prepared into at least 5 aqueous solutions of different concentrations, in vitro imaging was performed by means of the 3.0 T clinical MRI system, and a longitudinal relaxation time and transverse relaxation time ($T_1$, $T_2$) were measured (three samples Gd-PAA-PLA-1, Gd-PAA-PLA-2 and Gd-PAA-PLA-3 were subjected to a parallel test for three times). As shown in FIG. 38, the relationship of $1/T_i$ changing with the gadolinium concentration is drawn, and a slope of the fitted straight line is the $T_i$ relaxation rate (i = 1 or 2). It can be seen that in the 3.0 T MRI scanning system (Philips, Ingenia), the $r_1$ value of the gadolinium chelate Gd-PAA-PLA is 40.63 $\pm$ 1.17 mM$^{-1}$ s$^{-1}$, the $r_2$ value is 62.67 $\pm$ 0.91 mM$^{-1}$ s$^{-1}$, and $r_2/r_1$ is 1.54 $\pm$ 0.02. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PAA-PLA also has a lower $r_2/r_1$ ratio, showing a better MRI contrast effect.

## Example 33

Preparation of gadolinium chelate (Gd-PGA-PEG)

40 mL of polyglycolic acid-polyethylene glycol ($M_w$ = 2000) copolymer solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PGA-PEG finally.

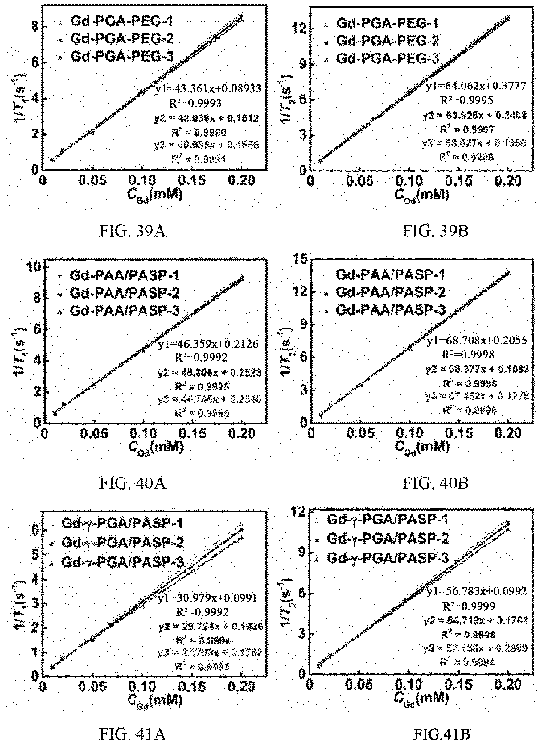

Characterization and performance tests were performed on a sample provided in Example 33. The gadolinium recovery rate of the sample prepared in Example 33 was 92.3%, indicating that the utilization rate of raw materials was higher. The sample prepared in Example 33 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PGA-PEG-1, Gd-PGA-PEG-2 and Gd-PGA-PEG-3 were subjected to a parallel test for three times). As shown in FIG. 39, the relationship of $1/T_i$ changing with the gadolinium concentration is drawn, and a slope of the fitted straight line is the $T_i$ relaxation rate (i = 1 or 2). It can be seen that in the 3.0 T MRI scanning system (Philips, Ingenia), the $r_1$ value of the gadolinium chelate Gd-PGA-PEG is 42.13 $\pm$ 1.19 mM$^{-1}$ s$^{-1}$, the $r_2$ value is 63.67 $\pm$ 0.56 mM$^{-1}$ s$^{-1}$, and $r_2/r_1$ is 1.51 $\pm$ 0.03. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PGA-PEG also has a lower $r_2/r_1$ ratio, showing a better MRI contrast effect.

## Example 34

Preparation of gadolinium chelate (Gd-PAA/PASP)

40 mL of polyacrylic acid/polyaspartic acid (1:1) (the $M_w$ of both are 2000) mixture solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-PAA/PASP finally.

Characterization and performance tests were performed on a sample provided in Example 34. The gadolinium recovery rate of the sample prepared in Example 34 was 92.9%, indicating that the utilization rate of raw materials was higher. The sample prepared in Example 34 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-PAA/PASP-1, Gd-PAA/PASP-2 and Gd-PAA/PASP-3 were subjected to a parallel test for three times). As shown in FIG. 40, the relationship of $1/T_i$ changing with the gadolinium concentration is drawn, and a slope of the fitted straight line is the $T_i$ relaxation rate (i = 1 or 2). It can be seen that in the 3.0 T MRI scanning system, the $r_1$ value of the gadolinium chelate Gd-PAA/PASP is 45.47 $\pm$ 0.82 mM$^{-1}$ s$^{-1}$, the $r_2$ value is 68.18 $\pm$ 0.65 mM$^{-1}$ s$^{-1}$, and $r_2/r_1$ is 1.50 $\pm$ 0.02. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-PAA/PASP also has a lower $r_2/r_1$ ratio, showing a better MRI contrast effect.

**Example 35**

Preparation of gadolinium chelate (Gd-γ-PGA/PASP)

40 mL of polyglutamic acid/polyaspartic acid (1:1) (the $M_w$ of both are 2000) mixture solution with a concentration of 4.0 mg/mL was prepared, and the polymer solution was heated at 100°C for refluxing. 0.8 mL of gadolinium nitrate solution with a concentration of 125 mM was immediately added to the reaction system, and the reaction continued for 60 minutes at 100°C under the magnetic stirring. After being cooled to the room temperature, the solution was purified by means of membrane dialysis to obtain a product labeled as Gd-γ-PGA/PASP finally.

Characterization and performance tests were performed on a sample provided in Example 35. The gadolinium recovery rate of the sample prepared in Example 35 was 82.8%, indicating that the utilization rate of raw materials was higher. The sample prepared in Example 35 was prepared into at least 5 aqueous solutions with different concentrations, in vitro imaging was performed by means of the 3.0 T clinical MRI system (Philips, Ingenia), and a longitudinal relaxation time and a transverse relaxation time ($T_1$, $T_2$) thereof were measured (three samples Gd-γ-PGA/PASP-1, Gd-γ-PGA/PASP-2 and Gd-γ-PGA/PASP-3 were subjected to a parallel test for three times). As shown in FIG. 41, the relationship of $1/Ti$ changing with the gadolinium concentration is drawn, and a slope of the fitted straight line is the $T_i$ relaxation rate (i = 1 or 2). It can be seen that in the 3.0 T MRI scanning system (Philips, Ingenia), the $r_1$ value of the gadolinium chelate Gd-γ-PGA/PASP is 29.47 $\pm$ 1.65 mM$^{-1}$ s$^{-1}$, the $r_2$ value is 54.55 $\pm$ 2.32 mM$^{-1}$ s$^{-1}$, and $r_2/r_1$ is 1.85 $\pm$ 0.03. The $r_1$ value is much higher than the $r_1$ value ($r_1$ = 4-7 mM$^{-1}$ s$^{-1}$) of the commercially available gadolinium-based contrast agent, and significantly higher than that of the highly toxic free gadolinium ion solution. In addition, the gadolinium chelate Gd-γ-PGA/PASP also has a lower $r_2/r_1$ ratio, showing a better MRI contrast effect.

The examples of the present disclosure are preferred embodiments, but the embodiments of the present disclosure are not limited to the foregoing specific examples. Any changes, modifications, substitutions, combinations, and simplifications thereof made without departing from the spirit and principles of the present disclosure should be equivalent alternatives and fall within the protection scope of the present disclosure.

**Claims**

1. A gadolinium chelate, which is a complex formed by chelation of gadolinium ions and a macromolecule, wherein the macromolecule comprises any one of or a copolymer or mixture of two or more of a carboxylic acid-containing high-molecular polymer, an amino-containing high-molecular polymer, a hydroxyl-containing high-molecular polymer, a polyester high-molecular polymer, a polyether high-molecular polymer, a polyamide high-molecular polymer, a protein, a polypeptide, or a polysaccharide.

2. The gadolinium chelate according to claim 1, wherein a molecular weight of the macromolecule is 1,000-1,000,000.

3. The gadolinium chelate according to claim 1, wherein the carboxylic acid-containing high-molecular polymer comprises any one or more selected from the group consisting of polyacrylic acid, polymaleic acid, polymethacrylic acid, poly(2-ethylacrylic acid), polyglutamic acid, and polyaspartic acid.

4. The gadolinium chelate according to claim 1, wherein the amino-containing high-molecular polymer comprises any one or more selected from the group consisting of polylysine, polyarginine, polyhistidine, and polyethyleneimine.

5. The gadolinium chelate according to claim 1, wherein the hydroxyl-containing high-molecular polymer comprises any one or more selected from the group consisting of polyserine, polythreonine, polytyrosine, and polyvinyl alcohol.

6. The gadolinium chelate according to claim 1, wherein the polyester high-molecular polymer comprises any one or more selected from the group consisting of polylactic acid, polyglycolic acid, polycaprolactone, and poly(2-hydroxy ethyl methacrylate).

7. The gadolinium chelate according to claim 1, wherein the polyether high-molecular polymer comprises any one or more selected from the group consisting of polyethylene glycol, polypropylene glycol, and polytetramethylene glycol.

8. The gadolinium chelate according to claim 1, wherein the polyamide high-molecular polymer comprises any one or more selected from the group consisting of poly glutamine, polyasparagine, polyacrylamide, and polymethacrylamide.

9. The gadolinium chelate according to claim 1, wherein the protein comprises any one or more selected from the group consisting of a human protein, an animal protein, a plant protein, and a recombinant protein.

10. The gadolinium chelate according to claim 1, wherein the polypeptide comprises any one or more selected from the group consisting of an RGD peptide and a β-amyloid polypeptide.

11. The gadolinium chelate according to claim 1, wherein the polysaccharide comprises any one or more of selected from the group consisting chitosan and sodium alginate.

12. A preparation method of the gadolinium chelate according to any one of claims 1-11, comprising: subjecting gadolinium ions and a macromolecule to a chelation reaction to obtain the gadolinium chelate.

13. An application of the gadolinium chelate according to any one of claims 1-11 in the preparation of a magnetic resonance imaging contrast agent.

FIG.1A                    FIG.1B

FIG. 2

FIG.3A

Gd-PAA, $C_{Gd}$=200μm

Gadavist, $C_{Gd}$=200μm

$H_2O$, $C_{Gd}$=0μm

FIG.3B

Gd-PAA, $C_{Gd}$=200μm

Gadavist, $C_{Gd}$=200μm

$H_2O$, $C_{Gd}$=0μm

FIG.3C

FIG. 4

FIG. 5A    FIG. 5B

FIG. 5C    FIG. 5D

y=55.649x+0.1797
R²=0.9959
y = 58.214x + 0.0795
R² = 0.9944
y=56.232x+0.1521
R²=0.9957

FIG. 6A

y=81.125x+0.2148
R²=0.9998
y = 79.933x + 0.199
R² = 0.9999
y=79.624x+0.188
R²=0.9994

FIG.6B

FIG. 7

FIG.8A

Gd-PASP, $C_{Gd}$=200μm

Gadavist, $C_{Gd}$=200μm

$H_2O$, $C_{Gd}$=0μm

Gd-PASP, $C_{Gd}$=200μm

Gadavist, $C_{Gd}$=200μm

$H_2O$, $C_{Gd}$=0μm

FIG.8B

Gd-PASP, $C_{Gd}$=200μm

Gadavist, $C_{Gd}$=200μm

$H_2O$, $C_{Gd}$=0μm

Gd-PASP, $C_{Gd}$=200μm

Gadavist, $C_{Gd}$=200μm

$H_2O$, $C_{Gd}$=0μm

239

5

FIG.8C

FIG. 9A

FIG. 9B

FIG. 10A

FIG. 10B

EP 4 317 206 A1

FIG. 11A

FIG. 11B

FIG. 12A

FIG. 12B

FIG. 13A

FIG. 13B

FIG. 14A

FIG. 14B

FIG. 15A

FIG. 15B

FIG. 16A

FIG. 16B

FIG. 17A

FIG. 17B

FIG. 18A

FIG. 18B

FIG. 19A

FIG. 19B

FIG. 20A

FIG. 20B

FIG. 21A

FIG. 21B

FIG. 22A

FIG. 22B

FIG. 23A

FIG. 23B

FIG. 24A

FIG. 24B

FIG. 25A

FIG. 25B

FIG. 26A

FIG. 26B

FIG. 27A

FIG. 27B

FIG. 28A

FIG. 28B

FIG. 29A

FIG. 29B

FIG. 30A

FIG. 30B

FIG. 31A

FIG. 31B

FIG. 32A

FIG. 32B

FIG. 33A

FIG. 33B

FIG. 34A

FIG. 34B

FIG. 35A

FIG. 35B

FIG. 36A

FIG. 36B

FIG. 37A

FIG. 37B

FIG. 38A

FIG. 38B

FIG. 39A

FIG. 39B

FIG. 40A

FIG. 40B

FIG. 41A

FIG.41B

## INTERNATIONAL SEARCH REPORT

| International application No. |
|---|
| **PCT/CN2021/127915** |

### A. CLASSIFICATION OF SUBJECT MATTER

C08F 8/42(2006.01)i; A61K 49/12(2006.01)i; A61K 49/14(2006.01)i; C08B 37/08(2006.01)i

According to International Patent Classification (IPC) or to both national classification and IPC

### B. FIELDS SEARCHED

Minimum documentation searched (classification system followed by classification symbols)

C08F; A61K; C08B

Documentation searched other than minimum documentation to the extent that such documents are included in the fields searched

Electronic data base consulted during the international search (name of data base and, where practicable, search terms used)

CNTXT; ENTXTC; DWPI; CNKI; Web of Science: 钆, 均聚物, 共聚物, 聚合物, 齐聚物, 高聚物, 大分子, 寡聚物, 高分子, 低聚物, 络合, 螯合, 配位, 配合, 聚甲基丙烯酸-2-羟乙酯, 聚苏氨酸, 聚丝氨酸, 乙基丙烯酸, 聚丙烯酸, 聚丁二醇, 聚酪氨酸, 聚马来酸, 壳聚糖, 聚甲基丙烯酸, 聚乙烯亚胺, 聚丙烯酰胺, 聚组氨酸, 聚甲基丙烯酰胺, 聚丙二醇, 聚乙醇酸, 动物蛋白, β-淀粉样多肽, 聚天冬酰胺, 海藻酸钠, RGD肽, 聚精氨酸, 人源蛋白, 聚乳酸, 聚谷氨酸, 重组蛋白, 聚己内酯, 聚谷氨酰胺, 聚天冬氨酸, 植物蛋白, 聚乙二醇, 聚赖氨酸, 聚乙烯醇, Gd, gadolinium, chelate, complex, coordinate, Polymethacrylic, Oligomer, Polyglutamic, Polyaspartic, Polymaleic, PGA, PASP, PPMA, PCL, PTMG, HPMA, polymer, PLA, copolymer, PPG, macromolecule, PMA, polyacrylic, PEAA, PAA, PEG, PMAA, PVA.

### C. DOCUMENTS CONSIDERED TO BE RELEVANT

| Category* | Citation of document, with indication, where appropriate, of the relevant passages | Relevant to claim No. |
|---|---|---|
| PX | CN 112940157 A (SOUTHERN MEDICAL UNIVERSITY) 11 June 2021 (2021-06-11) <br> claims 1-13 | 1-13 |
| PX | CN 113318243 A (SOUTHERN MEDICAL UNIVERSITY) 31 August 2021 (2021-08-31) <br> reference example 2 | 1-13 |
| X | CN 101401943 A (GUANGDONG UNIVERSITY OF TECHNOLOGY) 08 April 2009 (2009-04-08) <br> embodiment 1, and description, page 2, paragraph 3 | 1-13 |
| X | C. Ünaleroğlu et al. "Characterization and Magnetic Behavior of Cobalt(II) and Gadolinium(III) Polyacrylates" <br> *Journal of Applied Polymer Science,* Vol. 56, No. 10, 06 June 1995 (1995-06-06), ISSN: 0021-8995, <br> experiment part, paragraph 1, table 1 and solution 1 | 1-12 |

☑ Further documents are listed in the continuation of Box C.     ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 December 2021** | **23 December 2021** |

| Name and mailing address of the ISA/CN | Authorized officer |
|---|---|
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2021/127915**

### C. DOCUMENTS CONSIDERED TO BE RELEVANT

| Category* | Citation of document, with indication, where appropriate, of the relevant passages | Relevant to claim No. |
|---|---|---|
| Y | C. Ünaleroğlu et al. "Characterization and Magnetic Behavior of Cobalt(II) and Gadolinium(III) Polyacrylates" *Journal of Applied Polymer Science,* Vol. 56, No. 10, 06 June 1995 (1995-06-06), ISSN: 0021-8995, experiment part, paragraph 1, table 1 and solution 1 | 13 |
| Y | CN 101401943 A (GUANGDONG UNIVERSITY OF TECHNOLOGY) 08 April 2009 (2009-04-08) embodiment 1, and description, page 2, paragraph 3 | 13 |
| X | CN 103055328 A (ZHEJIANG UNIVERSITY) 24 April 2013 (2013-04-24) claims 1-8 | 1-13 |
| X | CN 103394101 A (ZHEJIANG UNIVERSITY) 20 November 2013 (2013-11-20) claims 1-5 | 1-13 |
| X | CN 1673252 A (EAST CHINA NORMAL UNIVERSITY) 28 September 2005 (2005-09-28) claims 1 and 2 | 1-13 |
| X | US 5466439 A (MAGNETIC RESEARCH INC.) 14 November 1995 (1995-11-14) embodiments 1-3 | 1-13 |
| X | US 2012058054 A1 (NA KUN et al.) 08 March 2012 (2012-03-08) claims 1-11, embodiments 1-2 | 1-13 |
| X | CN 111892645 A (SOUTHERN UNIVERSITY OF SCIENCE AND TECHNOLOGY) 06 November 2020 (2020-11-06) claims 1-9 | 1-13 |

Form PCT/ISA/210 (second sheet) (January 2015)

## INTERNATIONAL SEARCH REPORT
### Information on patent family members

| International application No. |
| --- |
| **PCT/CN2021/127915** |

| Patent document cited in search report | | | Publication date (day/month/year) | Patent family member(s) | | | Publication date (day/month/year) |
| --- | --- | --- | --- | --- | --- | --- | --- |
| CN | 112940157 | A | 11 June 2021 | None | | | |
| CN | 113318243 | A | 31 August 2021 | None | | | |
| CN | 101401943 | A | 08 April 2009 | CN | 101401943 | B | 13 April 2011 |
| CN | 103055328 | A | 24 April 2013 | CN | 103055328 | B | 29 October 2014 |
| CN | 103394101 | A | 20 November 2013 | CN | 103394101 | B | 29 October 2014 |
| CN | 1673252 | A | 28 September 2005 | CN | 100355806 | C | 19 December 2007 |
| US | 5466439 | A | 14 November 1995 | None | | | |
| US | 2012058054 | A1 | 08 March 2012 | US | 9072782 | B2 | 07 July 2015 |
| | | | | CN | 102803270 | A | 28 November 2012 |
| | | | | CN | 102803270 | B | 22 April 2015 |
| | | | | WO | 2010128787 | A2 | 11 November 2010 |
| | | | | WO | 2010128787 | A3 | 24 March 2011 |
| | | | | WO | 2010128787 | A9 | 12 May 2011 |
| | | | | KR | 20100120971 | A | 17 November 2010 |
| | | | | KR | 101059285 | B1 | 24 August 2011 |
| | | | | EP | 2447269 | A2 | 02 May 2012 |
| | | | | EP | 2447269 | A4 | 01 July 2015 |
| | | | | EP | 2447269 | B1 | 01 November 2017 |
| CN | 111892645 | A | 06 November 2020 | None | | | |

Form PCT/ISA/210 (patent family annex) (January 2015)